# EUROPEAN PATENT APPLICATION

(11) **EP 4 553 170 A1**
(43) Date of publication of application: **14.05.2025**
(21) Application number: 24165652.9
(22) Date of filing: 22.03.2024
(51) Int. Cl.: C12Q 1/6834

(54) **PELLET COMPRISING A SPECIFIC POLYMER SPECIES**

(30) Priority: 13.11.2023 EP 23209504
(71) Applicant: midge medical GmbH, 12099 Berlin (DE)
(72) Inventor: WAHNES, Christian, 12099 Berlin (DE); DIEBOLD, Michael, 12099 Berlin (DE)
(74) Representative: Eisenführ Speiser

(57) **Abstract**

The invention relates , in its categories, to a pellet, a method for detecting a target analyte, to the use of a pellet, to the use of poly-(2-ethyl-2-oxazolin) as a disintegrating agent, to the use of poly-(2-ethyl-2-oxazolin) for manufacturing a pellet, to the use of a pellet for producing a solution, to a method for producing a solution from a pellet, to a use of poly-(2-ethyl-2-oxazolin), to a method for manufacturing a pellet, to a test kit and to the use of a test kit.

## Description

The invention relates to a pellet - also called bead - to be used in test kits for detecting the presence of a target nucleic acid, for instance DNA or RNA in a sample, by way of isothermal nucleic acid amplification and fluorescence.

Nucleic acid amplification technologies are used to amplify the amount of a target nucleic acid in a sample in order detect such target nucleic acid in the sample. A known nucleic acid amplification technology is Polymerase Chain Reaction (PCR). Isothermal nucleic acid amplification technologies offer advantages over polymerase chain reaction (PCR) in that they do not require thermal cycling or sophisticated laboratory equipment.

Known isothermal nucleic acid amplification technologies are inter alia Recombinase Polymerase Amplification (RPA) and Strand Invasion Based Amplification (SIBA) and other methods known to persons skilled in the art.

Recombinase polymerase amplification (RPA) is a known method for amplifying the amount of a target analyte, in particular a nucleic acid such as DNA or RNA in a sample. For recombinase polymerase amplification three core enzymes are used: a recombinase, a single-stranded DNA-binding protein (SSB) and a strand-displacing polymerase. Recombinases can pair oligonucleotide primers with homologous sequences in duplex DNA. SSB binds to displaced strands of DNA and prevents the primers from being displaced. The strand-displacing polymerase begins DNA synthesis at sites where the primer has bound to the target DNA. Thus, if a target gene sequence is indeed present in the tested sample, an exponential DNA amplification reaction can be achieved to amplify a small amount of a target nucleic acid to detectable levels within minutes at temperatures between 37°C and 42°C.

The three core RPA enzymes can be supplemented by further enzymes to provide extra functionality. Addition of exonuclease III allows the use of an exo probe for real-time, fluorescence detection. Addition of endonuclease IV (Nfo) allows detection on lateral flow strips. If a reverse transcriptase that works at 37 to 42 °C is added then RNA can be reverse transcribed and the cDNA produced amplified all in one step.

By adding a reverse transcriptase enzyme to an RPA reaction, it can detect RNA as well as DNA, without the need for a separate step to produce cDNA. It is an advantage of RPA that it is isothermal and thus only requires simple equipment. While RPA operates best at temperatures between 37 °C and 42 °C it still works at room temperature.

For detecting the presence of a targeted nucleic acid in a sample, fluorescence detection technique can be used. After the light source at specific wavelength illuminates on the targeted nucleic acids, the DNA-binding dyes or fluorescein-binding probes of the nucleic acids will react and enable fluorescent signals to be emitted. The fluorescent signal is an indication of the existence of the targeted nucleic acids.

The present invention relates to a method for detecting a target analyte, in particular a fast and easy to handle method for isothermal amplification of nucleic acids, including DNA and RNA. Particularly, the invention relates to diagnostic methods for rapidly diagnosing, for example, at least two infectious agents, or at least two different targets in the same infectious agent, in a biological sample of interest. The invention also relates to the use of a pellet. The invention further relates to the use of poly-(2-ethyl-2-oxazolin) as a disintegrating agent. The invention also relates to the use of poly-(2-ethyl-2-oxazolin) for manufacturing a pellet. The invention further relates to the use of a pellet for producing a solution. The invention moreover relates to a method for producing a solution from a pellet. The invention further relates to the use of poly-(2-ethyl-2-oxazolin) in a method. The invention also relates to a method for manufacturing a pellet. The invention also relates to a test kit. The invention further relates to the use of a test kit.

Nucleic acid amplification techniques (NAATs), like molecular real-time PCR assays, are usually very sensitive, and specific but when it comes to time-to-result, PCR still has the inherent disadvantage to require highly-equipped laboratories and well-trained personnel. Therefore, new portable diagnostic solutions having a good specificity and sensitivity and being able to provide reliable results in situ at the place of testing are urgently needed.

Regarding nucleic acid-based preparative, cloning and diagnostic techniques, the development of polymerase chain reaction (PCR) in the 1980^{ies} by the later Nobel laureate Kary Mullis and team as rapid and reliable method to amplify DNA revolutionized molecular biology in general, as scientists suddenly had the opportunity to obtain millions of copies of a DNA target molecule of interest in short time. Simplicity and efficiency (e.g., nowadays possibilities to perform single-molecule/cell PCR) represent significant advantages of PCR techniques.

Still, PCR suffers from certain drawbacks, including the inherent need for iterative rounds of thermal cycling and the shift between different temperatures (repeated cycles of two or three temperature-dependent steps during the amplification process) and the use of high (>90°C) temperatures. These drawbacks have led to the development of alternative amplification methods.

An important class of PCR alternatives are so called isothermal amplification methods (for a review, see Zanoli and Spoto, Biosensors (Basel), 2012 3(1): 18-43)). The huge advantage over PCR is the fact that isothermal nucleic acid amplification methods are not requiring any thermal cycling at all, but can be conducted at constant temperatures. This makes the amplification process much easier to operate and to control. Further, less energy is needed than for PCR methods, the latter inherently requiring rapid heating and cooling steps. The constant temperature of isothermal methods additionally allows fully enclosed micro-structured devices into which performing the isothermal amplification reduces the risk of sample contamination and implies low sample consumption, multiplex DNA analysis, integration and portable devices realization. Finally, the constant temperature would be highly preferable for point-of-need and/or portable diagnostic devices, as recently developed by the present applicant (DE 10 2020 109 744.1, published as DE 10 2020 109 744 A1 which is incorporated herein by reference).

Isothermal amplification strategies available at date include nucleic acid sequence-based amplification (NASBA), loop-mediated isothermal amplification (LAMP), helicase-dependent amplification (HDA), rolling circle amplification (RCA), multiple displacement amplification (MDA), recombinase polymerase amplification (RPA) (see again: Zanoli and Spoto, 2012, *supra;* for a comprehensive survey on nowadays RPA techniques: Li et al., Analyst, 2019, 144, 31, pp. 31 to 67), or strand invasion based amplification (SIBA^{®} - SIBA in the following) (Hoser et al., PLoS ONE 9(11): e112656. doi:10.1371/journal.pone.0112656). Still, just as PCR isothermal technologies too, tend to produce non-specific amplification products as major drawbacks.

Whereas, for example, NASBA, RPA and HDA (and certain kinds of quantitative PCR) rely on the use of target-specific probes to provide for higher specificity of the reaction, LAMP uses additional primers instead of probes together with a strand displacement polymerase (see for example: Notomi et al., 2000, Nucleic Acids Research 28: e63). The use of numerous large primers in an at least 30 min incubation, however, has been shown to produce false-positive results - an outcome definitely to be avoided during diagnostic applications, particularly for diagnosing infectious diseases. Further, there is the problem that LAMP reactions require a high temperature of 65°C, which is unfavourable for diagnostic devices for use in a home environment due to regulatory and practical issues. Consequently, LAMP will not be the method of choice in case a diagnostic nucleic acid amplification result is needed in a short time in a handheld device, which has to fulfil certain regulatory requirements to be suitable for non-laboratory use.

RPA and SIBA technology both rely on the use of a recombinase during the binding and amplification process. Initially, a nucleoprotein complex constituted by oligonucleotide primers and the recombinase proteins is formed for RPA- and SIBA-type nucleic acid amplification strategies, these complexes facilitating the primer binding to the template DNA. Due to their short run times (around 15 min), unspecific amplification has not been observed. A certain advantage is the fact the recombinase can tolerate at least one incorrect base without preventing strand invasion necessary to start the reaction.

In parallel to the biochemical advancements in nucleic acid amplification, also the development of devices including suitable reaction and detection chambers for performing nucleic acid amplification in an optimized manner have steadily developed. A great trend has been the development of microfluidic devices and, in general, a trend of miniaturizing or "nano-tizing reaction chambers", i.e., putting them into nanoforms, has been of great interest to decrease the sample volumes and thus the amount of reagents needed and to achieve advantages in biochemical detection, time to result, faster thermal transfer, and to have the potential for automation and integration and, what is more, to have the possibility of multiplexed or multimeric tests. Multimeric in this case means multiplexing in the sense of parallel reactions being conducted in separate (locally distinct) reaction chambers.

Test system are known, were testing chambers or reaction chambers are prefilled with a mixture of chemicals including target-specific probes and enzymes that can cause an amplification of nucleic acid in a sample. For testing, a lysed sample needs to be added. The mixture of chemicals can be provided as a pellet or bead in the reaction chamber, see for instance WO 2021/204901 A1 or US 2019/0076841 A1.

It is an object of the invention to improve the reliability and the ease of use of test systems, in particular of test systems for detecting a target nucleic acid.

The inventors found that the reaction of the enzymes in the pellet and the target nucleic acid in the sampled may be affected by the physical, in particular mechanical structure of the pellet. Ideally, the pellet should disintegrate quickly once the sample is added. However, this is not always the case. Problems may be stray light caused by solid particles that remain in the reaction chamber, for instance the testing vial, an incomplete reaction or reduced reaction dynamics resulting in a measurement graph that is less significant.

The present invention is defined in the claims and described in detail hereinafter.

The present invention relates , in its categories, to a pellet, a method for detecting a target analyte, to the use of a pellet, to the use of poly-(2-ethyl-2-oxazolin) as a disintegrating agent, to the use of poly-(2-ethyl-2-oxazolin) for manufacturing a pellet, to the use of a pellet for producing a solution, to a method for producing a solution from a pellet, to a use of poly-(2-ethyl-2-oxazolin), to a method for manufacturing a pellet, to a test kit and to the use of a test kit. Embodiments, aspects or properties that are described in connection with one of these categories or described as preferred are each correspondingly or analogously applicable to the respective other categories, and vice versa.

Unless stated otherwise, preferred aspects or embodiments of the invention and their various categories can be combined with other aspects or embodiments of the invention and their various categories, especially with other preferred aspects or embodiments. The combination of respectively preferred aspects or embodiments with one another again results in preferred aspects or embodiments of the invention.

In a primary aspect of the present invention, the above-specified objectives are achieved and the above-specified problems are solved in whole or in part by a pellet, comprising:
- a first species of enzyme
- a polymeric species comprising a chemical structural element defined by the following formula (I):

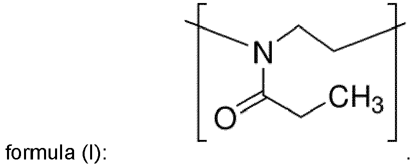

The term "pellet" is well known in the technical field of the present invention and is used herein in accordance with the usual understanding of the skilled person. In the technical field of the present invention, a pellet is frequently also termed "bead".

That the pellet comprises a species of enzyme means that the pellet comprises at least one first species of enzyme; more than one enzyme molecule of said species can be present as first species of enzyme.

The polymeric species comprising the chemical structural element defined by formula (I) can comprise further structural elements. Suitable polymeric species within the meaning of the present text are soluble in acetate buffer at 37 °C to an extent that a solution with polymer content of 7 wt.-% or more of the suitable polymer (polymeric species) in acetate buffer can be produced at 37°C and is stable at this temperature for at least 1 h.

The present invention, with its various aspects, especially and preferably relates to a pellet (as described above, preferably as identified above as preferred),
- wherein the pellet comprises one, two, three or more additional species of enzymes;
   and/or
- wherein the pellet additionally comprises adenosine triphosphate (ATP); and/or
- wherein the pellet additionally comprises deoxynucleotide triphosphate (dNTP) and/or dideoxynucleotide triphosphate (ddNTP);
   and/or
- wherein the pellet additionally comprises dithiothreitol (DTT);
   and/or
- wherein the pellet additionally comprises creatine phosphate (CP)
   and/or
- wherein the polymeric species comprising a chemical structural element defined by formula (I) is poly-(2-ethyl-2-oxazolin);
   and/or
- wherein the pellet contains poly-(2-ethyl-2-oxazolin) in an amount of 3 wt.-%to 8.5 wt.-%, preferably from 6 wt.-% to , more preferably from 6.5 wt.-% to 7.5 wt.-%, even more preferably in an amount of 7 wt.-%, with respect to the total dry mass of the pellet;
   and/or
- wherein the pellet contains poly-(2-ethyl-2-oxazolin) and
- wherein at least 90 % of the poly-(2-ethyl-2-oxazolin) molecules in the pellet, preferably at least 92 %, more preferably at least 95 % have an average relative molecular mass in the range of 10 000 to 100 000, preferably in the range of 15 000 and 80 000, more preferably in the range of 20 000 and 70 000.

That the pellet comprises one additional species of enzyme means that the pellet comprises a first species of enzyme and, additionally, one further species of enzyme) as second species of enzyme. That the pellet comprises two additional species of enzyme means that the pellet comprises a first species of enzyme and, additionally, two further species of enzyme as second and third species of enzyme respectively. That the pellet comprises three additional species of enzyme means that the pellet comprises a first species of enzyme and, additionally, three further species of enzyme as second, third and fourth species of enzyme respectively. That the pellet comprises more additional species of enzyme means that the pellet comprises a first species of enzyme and, additionally, four or more further species of enzyme as second, third, fourth, fifth and (where applicable) further species of enzyme respectively.

In many cases, especially in cases where the pellet is used in combination with isothermal nucleic acid amplification, it is preferred that the pellet additionally comprises adenosine triphosphate (ATP). In the field of the present invention, many benefits of adenosine triphosphate (ATP) are well-known to the skilled person. Providing adenosine triphosphate (ATP) to a process as part of a pellet according to the present invention in many cases allows for favourably low effort for dosing and is associated with a favourably fast procedure for carrying out isothermal nucleic acid amplification and subsequent detection; this is especially advantageous in non-laboratory use.

In many cases, especially in cases where the pellet is used in combination with isothermal nucleic acid amplification, it is preferred that the pellet additionally comprises deoxynucleotide triphosphate (dNTP) and/or dideoxynucleotide triphosphate (ddNTP). In the field of the present invention, many benefits of deoxynucleotide triphosphate (dNTP) and/or dideoxynucleotide triphosphate (ddNTP) are well-known to the skilled person. Providing of deoxynucleotide triphosphate (dNTP) and/or dideoxynucleotide triphosphate (ddNTP) to a process as part of a pellet according to the present invention in many cases allows for favourably low effort for dosing and is associated with a favourably fast procedure for carrying out isothermal nucleic acid amplification and subsequent detection; this is especially advantageous in non-laboratory uses.

In many cases, especially in cases where the pellet is used in combination with isothermal nucleic acid amplification, it is preferred that the pellet additionally comprises dithiothreitol (DTT). Dithiothreitol (DTT) (CAS Number 3483-12-3) is also known by the name "(2S,3S)-1,4-Bis(sulfanyl)butane-2,3-diol". In the field of the present invention, many benefits of dithiothreitol (DTT) are well-known to the skilled person. Providing of dithiothreitol (DTT) to a process as part of a pellet according to the present invention in many cases allows for favourably low effort for dosing and is associated with a favourably fast procedure for carrying out isothermal nucleic acid amplification and subsequent detection; this is especially advantageous in non-laboratory uses.

In many cases it is preferred that the polymeric species comprising a chemical structural element defined by formula (I) is poly-(2-ethyl-2-oxazolin). With Poly-(2-ethyl-2-oxazolin) the pellet can be dissolved in suitable solvents with especially favourable results. Moreover, in many cases pellets comprising poly-(2-ethyl-2-oxazolin) upon dissolution lead to solutions with entropic properties that are especially favourable for favourably good functionality of many enzymes. This is an advantage in processes utilising isothermal nucleic acid amplification and subsequent detection; in such processes, pellets comprising poly-(2-ethyl-2-oxazolin) are frequently preferred. These advantages are particularly well achieved when the pellet contains poly-(2-ethyl-2-oxazolin) in an amount of 3 wt.-%to 8.5 wt.-%, preferably from 6 wt.-% to , more preferably from 6.5 wt.-% to 7.5 wt.-%, even more preferably in an amount of 7 wt.-%, with respect to the total dry mass of the pellet.

The advantages of the present invention are particularly well achieved with a pellet (as described above, preferably as identified above as preferred) that contains poly-(2-ethyl-2-oxazolin) and wherein at least 90 % of the poly-(2-ethyl-2-oxazolin) molecules in the pellet, preferably at least 92 %, more preferably at least 95 % have an average relative molecular mass in the range of 10 000 to 100 000, preferably in the range of 15 000 and 80 000, more preferably in the range of 20 000 and 70 000.

The present invention, with its various aspects, especially and preferably relates to a pellet (as described above, preferably as identified above as preferred), wherein the one, two, three or more different species of enzyme are selected from the group consisting of:
- enzymes for recombinase-polymerase-amplification, including a recombinase, a single-stranded DNA-binding protein (SSB) and a strand-displacing polymerase,
   and, optionally, in addition at least one of the following enzymes: reverse transkriptase, creatine kinase, exonuclease III and endonuclease IV (Nfo).

With a pellet comprising these enzymes, the effects and advantages of the present invention are realised in a particularly favourable way, especially in cases where the pellet is used in test kits for detecting the presence of a target nucleic acid, for instance DNA or RNA in a sample, by way of isothermal nucleic acid amplification and fluorescence. The skilled person is aware of the properties and uses of reverse transkriptase, creatine kinase, exonuclease III and endonuclease IV (Nfo) and uses these enzymes based on the requirements of the individual case and in accordance with common general knowledge.

The present invention, with its various aspects, especially and preferably relates to a pellet (as described above, preferably as identified above as preferred), wherein
- the pellet does not have an extension of more than 20 mm, preferably more than 15 mm, more preferably more than 12 mm, even more preferably more than 10 mm in any direction of space;
   and/or
- the pellet does not have an extension of less than 4.6 mm, preferably less than 5 mm, more preferably less than 7 mm, even more preferably less than 9 mm in any direction of space;
   and/or
- the pellet has a weight in the range of 1 mg to 10 mg, preferably 2 mg to 8 mg, more preferably 5.5 mg to 7.8 mg, even more preferably 6.5 mg to 7.5 mg, yet more preferably 6.8 mg to 7.2 mg;
   and/or
- the pellet has a volume in the range of 1 µL to 100 µL, preferably 3 µL to 70 µL, more preferably 6 µL to 50 µL.

Pellets with an extension, weight and/or volume in the above defined ranges are particularly favourable for use in test kits. They present a favourable compromise between being large enough to handle and being able to be dissolved in small enough amounts of solvent to allow small sample volumes to be used in the respective test. Combinations of the above-defined weight ranges with the above-defined volume ranges result in pellets with a particularly preferred density in which small volume is favourably combined with high functionality of the substances comprised by the pellet after dissolution of the pellet in suitable solvent (preferably a solvent as described as preferred in the context of the present invention).

The present invention, with its various aspects, especially and preferably relates to a pellet (as described above, preferably as identified above as preferred),
- wherein the pellet is completely soluble in tetrahydrofuran, determined according to example B;
   and/or, preferably "and"
- wherein the pellet is essentially free of polyethylene glycol, preferably completely free of polyethylene glycol;
   and/or, preferably "and"
- wherein the pellet is essentially free of methylcellulose and/or carboxy-methylcellulose, preferably completely free of methylcellulose and/or carboxy-methylcellulose;
   and/or, preferably "and"
- wherein the pellet is essentially free of starch, preferably completely free of starch;
   and/or, preferably "and"
- wherein the pellet is essentially free of alginates, in particular sodium alginate, preferably completely free of alginates, in particular sodium alginate;
   and/or, preferably "and"
- wherein the pellet is essentially free of soja-polysaccharide, preferably completely free of soja-polysaccharide;
   and/or, preferably "and"
- wherein the pellet is essentially free of poly methacrylic acid gel, prefer-ably completely free of poly methacrylic acid gel;
   and/or, preferably "and"
- wherein the pellet is essentially free of Poly[1-(4-sulfophenyl)eth-ylen], prefer-ably completely free of Poly[1-(4-sulfophenyl)eth-ylen];
   and/or, preferably "and"
- wherein the pellet is essentially free of bicarbonates, prefer-ably completely free of bicarbonates;
   and/or, preferably "and"
- wherein the pellet is essentially free of substances that produce gaseous substances upon contact with acetate buffer solutions;
   and/or, preferably "and"
- wherein the pellet is essentially free of microgels, preferably completely free of microgels.

In many cases the pellet is particularly suitable for use in test kits, in particular non-laboratory test-kits, when the pellet is soluble in tetrahydrofuran, determined according to example B.

Polyethylene glycol is also known by the alternative terms "poly(oxyethylene)" and "polyethylene oxide)".

The use of polyethylene glycol in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of methylcellulose and/or carboxy-methylcellulose in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of starch in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of alginates, in particular sodium alginate, in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of soja-polysaccharide in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of poly methacrylic acid gel in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of poly[1-(4-sulfophenyl)ethylen] in pellets for test kits is frequently associated with disadvantageous dissolution behaviour of pellets, unfavourably high turbidity of solutions obtained from the dissolution of such pellets and low reproducibility of the dissolution behaviour of the respective pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of bicarbonates in pellets for test kits is frequently associated with disadvantageous dissolution behaviour of pellets, unfavourably high turbidity of solutions obtained from the dissolution of such pellets and low reproducibility of the dissolution behaviour of the respective pellets. It is especially disadvantageous that bicarbonates upon dissolution in acetate buffer react to result in gaseous products; such gaseous products are frequently highly problematic in the detection of fluorescence signals in solutions obtained from such pellets. It is therefore also preferred that the pellet is free from bicarbonates and free from other substances that produce gaseous substances upon contact with acetate buffer solutions. Other substances produce gaseous substances. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The use of microgels in pellets for test kits is frequently associated with disadvantageous dissolution behaviour of pellets and unfavourably high turbidity of solutions obtained from the dissolution of such pellets. These are severe disadvantages especially when the pellet is used as part of a test kit in particular in test kits for detecting the presence of a target nucleic acid, for instance DNA or RNA in a sample, by way of isothermal nucleic acid amplification and fluorescence.

The invention also relates to a method for detecting a target analyte comprising the following steps:

| | |
|---|---|
| M1) | providing a pellet (as described above, preferably as identified above as preferred); |
| M2) | providing an acetate buffer; |
| M3) | providing, preferably as ingredient of the pellet, at least one single-strand binding protein, at least one, and preferably at least two target sequence specific primers, at least one DNA polymerase, and optionally: providing at least one reverse transcriptase; |
| M4) | providing at least one biological sample to be analysed for the presence of the at least one target sequence, and optionally providing an extraction kit, preferably a sterile extraction kit, to obtain the biological sample; |
| M5) | optionally: providing at least one probe and optionally providing, preferably as constituent of the pellet, at least one enzyme activating the probe so that a detectable signal is generated; |
| M6) | dissolving the pellet provided in step M1) in the acetate buffer solution provided in step M2) so that a solution of the ingredients of the pellet in the acetate buffer solution results; |
| M7) | initiating the amplification reaction by adding at least one starting reagent, preferably wherein the starting reagent comprises at least one metal oxide, more preferably at least one metal oxide nano-particle, including zinc oxide, and/or by contacting the components of (a), (b) and optionally (c); and |
| M8) | obtaining the at least one amplified target sequence and/or obtaining at least one detectable signal each signal being indicative of a successful amplification of the respective target sequence amplified. |

With the above method for detecting a target analyte the effects advantages of the present invention, specifically also the advantages described in combination with the pellet according to the present invention, are achieved in a particularly favourable way.

The present invention, with its various aspects, especially and preferably relates to a method (as described above, preferably as identified above as preferred),
- wherein the method for detecting a target analyte is an isothermal method of nucleic acid amplification of at least one target sequence, preferably in a biological sample;
   and/or
- wherein the solution of the ingredients of the pellet in the acetate buffer solution resulting in step M6) contains an amount of poly-(2-ethyl-2-oxazolin) in the range of 30 vol.-% to 95 vol.-%, preferably 40 vol.-% to 92 vol.-%, more preferably 50 vol.-% to 90 vol.-%, with regard to the total volume of the solution;
   and/or
- wherein the solution of the ingredients of the pellet in the acetate buffer solution resulting in step M6) contains adenosine triphosphate (ATP) in a concentration in the range of 1 mM to 4 mM, preferably 2 mM to 3 mM, more preferably 2.2 mM to 2.8 mM, yet more preferably 2.3 mM to 2.6 mM, even more preferably in a concentration of 2.5 mM;
   and/or
- wherein the solution of the ingredients of the pellet in the acetate buffer solution resulting in step M6) contains deoxynucleotide triphosphate (dNTP) and/or dideoxynucleotide triphosphate (ddNTP) in a concentration in the range of 100 µM to 800 µM, preferably 250 µM to 600 µM, more preferably 350 µM to 550 µM, yet more preferably 400 µM to 500 µM, even more preferably in a concentration of 450 µM;
   and/or
- wherein the solution of the ingredients of the pellet in the acetate buffer solution resulting in step M6) contains dithiothreitol (DTT) in a concentration in the range of 1 mM to 3 mM, preferably 1.5 mM to 2.7 mM, more preferably 1.6 mM to 2.5 mM, yet more preferably 1.8 mM to 2.2 mM, even more preferably in a concentration of 2 mM;
   and/or
- wherein the solution of the ingredients of the pellet in the acetate buffer solution resulting in step M6) contains creatine phosphate (CP) in a concentration in the range of 10 mM to 100 mM, preferably 20 mM to 70 mM, more preferably 40 mM to 60 mM, yet more preferably 45 mM to 55 mM, even more preferably in a concentration of 50 mM.

The effects and advantages discussed herein with regard to the pellet of the present invention are particularly well achieved when the method (according to the present invention) for detecting a target analyte is an isothermal method of nucleic acid amplification of at least one target sequence, preferably in a biological sample.

When the solution of the ingredients of the pellet in the acetate buffer solution resulting in step M6) contains the above defined amounts of poly-(2-ethyl-2-oxazolin) the solution has particularly favourable entropic properties that are especially favourable for favourably good functionality of many enzymes. This is especially advantageous in the method according to the present invention.

When the solution of the ingredients of the pellet in the acetate buffer solution resulting in step M6) contains adenosine triphosphate (ATP) in the above defined concentrations, the handling of ATP in the method of the present invention is favourably efficient and simple with favourably little time needed for ATP dosing at the point where the method is carried out. This is particularly favourably if the method is carried out in a non-laboratory setting. In the field of the present invention, many benefits of adenosine triphosphate (ATP) are well-known to the skilled person and ATP is used in accordance with this knowledge.

When the solution of the ingredients of the pellet in the acetate buffer solution resulting in step M6) contains deoxynucleotide triphosphate (dNTP) and/or dideoxynucleotide triphosphate (ddNTP) in the above defined concentrations, the effects and advantages associated with the method of the present invention are achieved particularly well. In the field of the present invention, many benefits of deoxynucleotide triphosphate (dNTP) and/or dideoxynucleotide triphosphate (ddNTP) are well-known to the skilled person and dNTP and ddNTP are used in accordance with this knowledge.

When the solution of the ingredients of the pellet in the acetate buffer solution resulting in step M6) contains dithiothreitol (DTT) in a concentration as defined above, the effects and advantages associated with the method of the present invention are achieved particularly well. In the field of the present invention, many benefits of dithiothreitol (DTT) are well-known to the skilled person and DTT is used in accordance with this knowledge.

The invention also relates to a use of a pellet (as described above, preferably as identified above as preferred), in a method (as described above, preferably as identified above as preferred).

With the use of the pellet according to the present invention in the method according to the present invention the effects and advantages associated with the different aspects of the present invention are achieved to a particularly favourable extent.

The invention also relates to a use of poly-(2-ethyl-2-oxazolin) as a disintegrating agent in a pellet (preferably as described above, preferably as identified above as preferred).

With the use of poly-(2-ethyl-2-oxazolin) as a disintegrating agent in a pellet, preferably a pellet according to the present invention, many of the effects and advantages associated with the pellet according to the present invention are achieved to a particularly favourable extent. With the use of poly-(2-ethyl-2-oxazolin) as disintegrating agent in the pellet, the pellet can be dissolved in suitable solvents with especially favourable results. Moreover, in many cases pellets comprising poly-(2-ethyl-2-oxazolin), upon dissolution, lead to solutions with entropic properties that are especially favourable for favourably good functionality of many enzymes. This is an advantage in processes utilising isothermal nucleic acid amplification and subsequent detection.

The invention also relates to a use of poly-(2-ethyl-2-oxazolin) for manufacturing a pellet (preferably as described above, preferably as identified above as preferred).

The use of poly-(2-ethyl-2-oxazolin) for manufacturing a pellet, preferably a pellet according to the present invention, results in a pellet, preferably in a pellet according to the present invention, that displays the effects and advantages associated with the present invention in a particularly favourable way.

The invention also relates to a use of a pellet (as described above, preferably as identified above as preferred) for producing a solution
- wherein the resulting solution contains an amount of poly-(2-ethyl-2-oxa-zolin) in the range of 30 vol.-% to 95 vol.-%, preferably 40 vol.-% to 92 vol.-%, more preferably 50 vol.-% to 90 vol.-%, with regard to the total volume of the solution;
   and/or
- wherein the resulting solution contains adenosine triphosphate (ATP) in a concentration in the range of 1 mM to 4 mM, preferably 2 mM to 3 mM, more preferably 2.2 mM to 2.8 mM, yet more preferably 2.3 mM to 2.6 mM, even more preferably in a concentration of 2.5 mM;
   and/or
- wherein the resulting solution contains deoxynucleotide triphosphate (dNTP) in a concentration in the range of 100 µM to 800 µM, preferably 250 µM to 600 µM, more preferably 350 µM to 550 µM, yet more preferably 400 µM to 500 µM, even more preferably in a concentration of 450 µM;
   and/or
- wherein the resulting solution contains adenosine triphosphate (ATP) in a concentration in the range of 1 mM to 3 mM, preferably 1.5 mM to 2.7 mM, more preferably 1.6 mM to 2.5 mM, yet more preferably 1.8 mM to 2.2 mM, even more preferably in a concentration of 2 mM.

The effects and advantages discussed in relation to the above features for other aspects of the present invention are achieved in a particularly positive way with the use of a pellet (as described above, preferably as identified above as preferred) for producing a solution. Especially the positive effects and advantages discussed herein with regard to solutions are achieved particularly well.

The invention also relates to a method for producing a solution from a pellet (as described above, preferably as identified above as preferred), wherein the method comprises the following steps:
S1) Supplying in different containers
   - a pellet
   - supplying an acetate buffer solution, preferably 10 µL to 100 µL of an acetate buffer solution, more preferably 20 µL to 80 µL, even more preferably 40 µL to 60 µL, yet more preferably 50 µL, as a solvent;
S2) contacting
   - the pellet supplied in Step S1)
      and
   - the solvent supplied in step S1)
      in a container so that the pellet supplied in step S1) dissolves in the solvent supplied in step S1) so that a solution results.

The use (as described above, preferably as identified above as preferred) of the pellet (as described above, preferably as identified above as preferred) for producing a solution is particularly effective when the use occurs according to the above method. The effects and advantages discussed herein with regard to pellets, solutions, methods and uses are achieved.

That a solution results means that the pellet is completely dissolved and no suspended particles or solid residue are visible to the unaided eye.

The present invention, with its various aspects, especially and preferably relates to a method (as described above, preferably as identified above as preferred),
- wherein the solvent is an aqueous solution comprising
   - potassium acetate, preferably in a concentration in the range of from 50 mM to 200 mM, more preferably 70 mM to 150 mM, even more preferably 90 mM to 120 mM, yet more preferably in a concentration of 95 mM to 105 mM;
      and/or
   - magnesium acetate, preferably in a concentration in the range of from 5 mM to 30 mM, more preferably 10 mM to 20 mM, even more preferably 11 mM to 17 mM, yet more preferably in a concentration of 13 mM to 15 mM;
      and/or
   - Tris acetate, preferably in a concentration in the range of from 10 mM to 50 mM, more preferably 30 mM to 40 mM, even more preferably 32 mM to 38 mM, yet more preferably in a concentration of 34 mM to 36 mM;
      and/or
   - a detergent or a detergent mixture, preferably in a concentration in the range of from 10 mM to 50 mM, more preferably 30 mM to 40 mM, even more preferably 32 mM to 38 mM, yet more preferably in a concentration of 34 mM to 36 mM;
      and/or
   - polyvinylsulfonic acid (PVSA), preferably in an amount of from 0.0001 wt.-% to 0.005 wt.-%, more preferably 0.0005 wt.-% to 0.003 wt.-%, even more preferably 0.0008 wt.-% to 0.0015 wt.-%, yet more preferably in a concentration of 0.0009 wt.-% to 0.0011 wt.-%;
      and/or
- wherein the solvent is essentially free of poly-(2-ethyl-2-oxazolin) and/or polyethylene glycol; preferably completely free of poly-(2-ethyl-2-oxazolin) and/or polyethylene glycol; more preferably essentially free of synthetic polymers;
   and/or
- wherein from the start of the contacting in step S2) until the resulting of a solution in step S2) no purposeful input of mechanical energy takes place;
   and/or
- wherein from the start of the contacting in step S2) until the resulting of a solution in step S2) the temperature never exceeds 45 °C, preferably never exceeds 38 °C, more preferably never exceeds 30 °C;
   and/or
- wherein from the start of the contacting in step S2) until the resulting of a solution in step S2) the temperature is never below 20 °C, preferably never below 18 °C, more preferably never below 15 °C.

That from the start of the contacting in step S2) until the resulting of a solution in step S2) no purposeful input of mechanical energy takes place means that for example no stirring, no shaking and other means of mechanically assisting dissolution like ultrasound etc. are applied.

That from the start of the contacting in step S2) until the resulting of a solution in step S2) the temperature never exceeds 45 °C, preferably never exceeds 38 °C, more preferably never exceeds 30 °C refers especially also to the temperatures of pellet, solvent, and (iii) container.

That from the start of the contacting in step S2) until the resulting of a solution in step S2) the temperature is never below 20 °C, preferably never below 18 °C, more preferably never below 15 °C refers especially also to the temperatures of pellet, solvent, and (iii) container.

The effects and advantages discussed in relation to the above features for other aspects of the present invention are achieved in a particularly positive way with the above method for producing a solution from a pellet. Especially the positive effects and advantages discussed herein with regard to solutions are achieved particularly well.

The invention also relates to a use of poly-(2-ethyl-2-oxazolin) to alter enzyme activity, preferably to improve enzyme activity, more preferably to improve enzyme activity in a method (as described above, preferably as identified above as preferred).

The use of poly-(2-ethyl-2-oxazolin) (as described above, preferably as identified above as preferred) leads to especially positive results as the effects are achieved in advantageous combination with the positively high solubility of poly-(2-ethyl-2-oxazolin) in aqueous solutions, especially in buffer solutions. In this use, enzyme activity is managed by managing excluded volume via the presence of poly-(2-ethyl-2-oxazolin).

The invention also relates to a method for manufacturing a pellet (preferably as described above, preferably as identified above as preferred), comprising at least the following steps:
G1) providing, preferably providing in separate containers, more preferably in separate containers and in defined amounts,
   - at least a first species of poly-(2-ethyl-2-oxazolin)
      and
      - at least one single-strand binding protein, at least one DNA polymerase, and optionally: at least one reverse transcriptase, together with suitable reaction components,
         and/or, preferably "and"
      - at least one, preferably at least two target sequence specific primers;
         and/or, preferably "and"
   - suitable reaction components, including at least one co-factor for at least one enzyme, dNTPs or a mixture of dNTPs and ddNTPs;
G2) intermixing the materials provided in step G1) within a common container so that a mixture comprising poly-(2-ethyl-2-oxazolin) results;
G3) pelletizing of the mixture comprising poly-(2-ethyl-2-oxazolin) resulting in step G2) so that a pellet results;
and, preferably, comprising the following additional step:
   G4) freeze-drying the pellet resulting in step G3 so that a freeze-dried pellet results.

That substances are supplied defined amounts means that the amounts defined beforehand i.e. according to a recipe.

The intermixing in step G2) of the materials provided in step G1) relates to the defined amounts of the materials that are provided in step G1).

The pellet according to the present invention is manufactures in a particularly efficient way and with particularly positive results with the method for manufacturing a pellet according to the present invention.

The present invention, with its various aspects, especially and preferably relates to a method (as described above, preferably as identified above as preferred),
- wherein the molecules of the first species of poly-(2-ethyl-2-oxazolin) provided in step G1) have an average relative molecular mass
   - in the range of 25000 Da
      and/or
   - a polydispersity index in the range of 1 to 2, preferably in the range of 1.2 to 1.6;
      or
- wherein the molecules of the first species of poly-(2-ethyl-2-oxazolin) provided in step G1) have an average relative molecular mass
   - in the range of 50000 Da
      and/or
   - a polydispersity index in the range of 2 to 5, preferably in the range of 3 to 4;
      and/or
- wherein, additionally, in step G1) at least a second species of poly-(2-ethyl-2-oxazolin) is provided, preferably in a separate container and more preferably in a separate container and in a defined amount, and
   - wherein the molecules of the second species of poly-(2-ethyl-2-oxazolin) provided in step G1) have an average relative molecular mass
   - in the range of 25000
      and/or
   - a polydispersity index in the range of 1 to 2, preferably in the range of 1.2 to 1.6;
      or
   - wherein the molecules of the second species of poly-(2-ethyl-2-oxazolin) provided in step G1) have an average relative molecular mass
   - in the range of 50000 Da
      and/or
   - a polydispersity index in the range of 2 to 5, preferably in the range of 3 to 4.

In many cases, a method (as described above, preferably as identified above as preferred) is preferred,
- wherein the molecules of the first species of poly-(2-ethyl-2-oxazolin) provided in step G1) have an average relative molecular mass
   - in the range of 25000 Da
      and/or
   - a polydispersity index in the range of 1 to 2, preferably in the range of 1.2 to 1.6;
      and
- wherein the molecules of the second species of poly-(2-ethyl-2-oxazolin) provided in step G1) have an average relative molecular mass
   - in the range of 50000 Da
      and/or
   - a polydispersity index in the range of 2 to 5, preferably in the range of 3 to 4.

In many cases, particularly positive results are achieved when the different species of poly-(2-ethyl-2-oxazolin) are used in the method as defined above. Especially the entropic properties of solutions made from pellets manufactured according to this method are highly desirable.

The present invention, with its various aspects, especially and preferably relates to a method (as described above, preferably as identified above as preferred),
- wherein
   - the molecules of the first species of poly-(2-ethyl-2-oxazolin) provided in step G1) have
      - an average relative molecular mass in the range of 25000 Da
         and/or
      - a polydispersity index in the range of 1 to 2, preferably in the range of 1.2 to 1.6;
         and
   - wherein, additionally, in step G1) at least a second species of poly-(2-ethyl-2-oxazolin) is provided, preferably in a separate container and more preferably in a separate container and in a defined amount, and
      - wherein the molecules of the second species of poly-(2-ethyl-2-oxazolin) provided in step G1) have
         - an average relative molecular mass in the range of 50000 Da
            and/or
         - a polydispersity index in the range of 2 to 5, preferably in the range of 3 to 4;
            and/or;
- wherein, additionally, in step G1) at least a second species of poly-(2-ethyl-2-oxazolin) is provided, preferably in a separate container and more preferably in a separate container and in a defined amount, and
   - the ratio of
      - the total mass of the second species of poly-(2-ethyl-2-oxazolin) provided in step G1) and/or present during intermixing in step G2)
         to the
      - the total mass of the first species of poly-(2-ethyl-2-oxa-zolin) provided in step G1) and/or present during intermixing in step G2)
      is in the range of 10 to 1.2, preferably 8 to 1.4, more preferably the weight ratio is 5.4 : 1.6.

In many cases, particularly positive results are achieved when the different species of poly-(2-ethyl-2-oxazolin) are used in the above-defined amounts. Especially the entropic properties of solutions made from pellets manufactured according to this method are most highly desirable in many of these cases.

The present invention, with its various aspects, especially and preferably relates to a method (as described above, preferably as identified above as preferred), wherein at least two pellets, a first pellet and a second pellet, are produced with the same overall composition, and wherein
the scattering intensity of a solution of the first whole pellet, determined according to steps ES1 and ES2 of example A, does not deviate by more than 30 %, preferably by not more than 20 %, particularly preferably by not more than 10 %, from the average scattering intensity determined from five arbitrarily selected fragments of the second pellet according to steps ES3 to ES5 of example A.

This indicates that the distribution of scattering substances, especially also poly-(2-ethyl-2-oxazolin) molecules, comprised by the pellet is sufficiently homogeneous in the whole pellet. A homogeneous distribution of scattering substances, especially poly-(2-ethyl-2-oxa-zolin) molecules, in the pellet is preferred, as such a homogeneous distribution in many cases leads to favourable dissolution characteristics of the pellet in suitable solvent (preferably a solvent as described as preferred in the context of the present invention). Further, such homogeneous distribution in many cases allows for particularly reproducible results when the pellet is used as part of a test kit in particular in test kits for detecting the presence of a target nucleic acid, for instance DNA or RNA in a sample, by way of isothermal nucleic acid amplification and fluorescence. The further effects and advantages of the present invention are achieved.

The invention also relates to a test kit comprising:
(i) a pellet (as described above, preferably as identified above as preferred);
(ii) at least one single-strand binding protein, at least one DNA polymerase, and optionally: at least one reverse transcriptase, together with suitable reaction components,
(iii) at least one, preferably at least two target sequence specific primers;
(iv) suitable reaction components, including at least one co-factor for at least one enzyme, dNTPs or a mixture of dNTPs and ddNTPs, at least one buffer system, and at least one reducing agent;
(v) at least one sterile set for extracting a biological probe to be analyzed and comprising at least one component configured to be applied to a system for analyzing the probe;
wherein preferably the test kit additionally comprises:
(vi) at least one probe and optionally at least one enzyme activating the probe;
   and/or
(vii) a second part comprising (i) to (v), preferably (i) to (vi), wherein the second part comprises a predetermined second target sequence acting as positive control and at least one, preferably at least two primers specific for the second target sequence, and preferably a second probe.

The method for detecting a target analyte (as described above, preferably as identified above as preferred) and the method for producing a solution from a pellet (as described above, preferably as identified above as preferred) are carried out in a particularly efficient way with the test kit according to the invention. The effects and advantages discussed in relation to other aspects of the present invention are particularly well achieved with the test kit of the present invention. The uses of the present invention also lead to especially positive results when the test kit according to the invention is employed in the respective use.

In many cases it is preferred that the substances defined under (ii) to (iv) above are part of the pellet.

The invention also relates to the use of at least kit (as described above, preferably as identified above as preferred) for detecting an analyte, preferably for performing an isothermal method of nucleic acid amplification of at least one target sequence.

When the test kit is used as described above very advantageous results are achieved. The effects and advantages discussed above in relation to other aspects of the present invention are also achieved.

The invention is described in more detail below by means of examples.

### Example A: Determining homogeneity of the distribution of scattering substances within pellets

ES1 A first whole pellet according to the present invention, weighing 7 mg, is dissolved in 50 µL of a buffer solution at 23°C, wherein the buffer solution contains potassium acetate in a concentration of 100 mM, magnesium acetate in a concentration of 14 mM, tris acetate in a concentration of 35 mM and a suitable non-ionic protein detergent; a solution of the whole pellet in buffer results;
the solution of the whole pellet in buffer is vortexed for 25 seconds and then stored for 10 hours in the dark at 23°C;
thereafter, the solution is vortexed again and then passed through a syringe filter with a pore size of 2 micrometers so that a filtered solution of the whole pellet in buffer results;

ES2 the scattering intensity (static light scattering) of the filtered solution of the whole pellet in buffer (resulting in step ES1) is determined at suitable dilution using a Zetasizer Ultra (commercially available from Malvern Panalytical Ltd, UK) with a laser wavelength of 633 nm;

ES3 a second whole pellet (same overall composition as the pellet of step ES1) according to the present invention, weighing 7 mg, is arbitrarily divided in at least five fragments of suitable size; five of said at least five fragments of the whole pellet are weighed and then individually dissolved in an individual amount of a buffer solution at 23°C, wherein each amount of buffer solution contains potassium acetate in a concentration of 100 mM, magnesium acetate in a concentration of 14 mM, tris acetate in a concentration of 35 mM and a suitable non-ionic protein detergent; and
wherein the solutions are each prepared to the same ratio of
   (i) weight of pellet ingredients
      to
   (ii) weight of buffer solution
as the solution of the whole pellet (first pellet) in buffer resulting in step ES1;
the five resulting solutions of the five pellet fragments in buffer are then each vortexed for 25 seconds and then stored for 10 hours in the dark at 23°C;
thereafter, the solutions are each vortexed again and then each passed through a syringe filter with a pore size of 2 micrometers so that five filtered solutions of a pellet fragments in buffer result;

ES4 the scattering intensity (static light scattering) of each of the five filtered solutions of the pellet fragments in buffer is determined using a Zetasizer Ultra (commercially available from Malvern Panalytical Ltd, UK), using the same conditions and parameters (specifically also the same dilution) as in step ES2;

ES5 the average scattering intensity is determined, for the five individual scattering intensity determined in step ES4, by summing the five individual scattering intensities and dividing the resulting sum by five;

ES6 the deviation, in percent, of the average scattering intensity resulting in step ES5 from the scattering intensity determined in step ES2, is determined.

### Example B: Determining solubility in tetrahydrofuran

To determine if a pellet is soluble in tetrahydrofuran, within the meaning of the present invention, the following test is carried out:
A whole pellet according to the present invention, weighing 7 mg, is dissolved in 50 µL of tetrahydrofuran at 23°C; the solution of the whole pellet in tetrahydrofuran is vortexed for 25 seconds and then stored for 10 hours in the dark at 23°C; thereafter, the solution is vortexed again; if before and/or after vortexing, the solution contains no suspended matter visible to the unaided eye and shows no sign of turbidity, visible to the unaided eye, then the pellet is soluble in tetrahydrofuran within the meaning of the present invention.

### Example 1:

### 1-1: Manufacture of pellet

In a first container a defined amount of a first species of poly-(2-ethyl-2-oxazolin) with an average Mₙ of 25 000 and a polydispersity index (PDI) of ≤1.4 was provided (obtained from Sigma-Aldrich).

In a second container, a defined amount of a second species of poly-(2-ethyl-2-oxazolin) with an average Mₙ of ~50 000 and a polydispersity index (PDI) of 3 to 4 was provided (obtained from Sigma-Aldrich).

The ratio of the total mass of the defined amount of the second species of poly-(2-ethyl-2-oxazolin) with an average Mₙ of ~50 000 (in the second container) to the total mass of the defined amount of the first species of poly-(2-ethyl-2-oxazolin) with an average Mₙ of 25 000 (in the first container) was 5.4 : 1.6.

The total mass of the defined amount of the second species of poly-(2-ethyl-2-oxazolin) with an average Mₙ of ~50 000 provided in the second container and the total mass of the defined amount of the first species of poly-(2-ethyl-2-oxazolin) with an average Mₙ of 25 000 provided in the first container, were placed together in a third container an mixed together by vortexing.

In a fourth container, a defined amount of a RB69 GP32 single strand binding protein (https://www.uniprot.orq/uniprot/P09797) with a molecular mass of 33.5 kDa was provided.

In a fifth container, a defined amount of a B. *subtillis* strand displacement polymerase (BSU https://www.uniprot.orq/uniprot/034996; Truncated version lacking 5'→3' exonuclease activity) with a molecular mass of 99 kDa was provided.

In a sixth container, a defined amount of a T6 Recombinase (UvsX H66S; https://www.uni-prot.org/uniprot/A0A346FJC7; Mutation at position 66 (H→S)) with a molecular mass of 44.1 kDa was provided.

In an seventh container, a defined amount of a T4 Coenzyme of Recombinase (UsvY; https://www.uniprot.orq/uniprot/P04537) with a molecular mass of 15.8 kDa was provided. In an eighth container, a defined amount of E.coli Exonuclease III (Exo III; https://www.uni-prot.orq/uniprot/P09030) with a molecular mass of 30.9 kDa was provided.

In an ninth container, a defined amount of creatine kinase (CK (Bovine) type B; https://www.uniprot.org/uniprotkb?query=Creatine+Kinase) with a molecular mass of 42.7 kDa was provided.

In a tenth container, a defined amount of adenosine triphosphate (ATP) was provided.

In an eleventh container, a defined amount of deoxynucleotide triphosphate (dNTP) was provided.

In a twelfth container, a defined amount of creatine phosphate (CP) was provided.

In a thirteenth container, a defined amount of dithiothreitol (DTT) was provided.

The amounts provided in the third, fourth, fifth, sixth, seventh, eighth, ninth, tenth, eleventh, twelfth and thirteenth container were placed together in an fourteenth container and thoroughly intermixed in the fourteenth container so that a mixture comprising poly-(2-ethyl-2-oxazolin) resulted and said mixture contained an amount of 7 wt.-% of poly-(2-ethyl-2-oxa-zolin) with regard to the total mass of said mixture.

A subset of said mixture was pelletized so that a first pellet with a mass of 7 mg resulted, said first pellet contained an amount of 7 wt.-% of poly-(2-ethyl-2-oxazolin) with regard to the total mass of said first pellet.

### 1-2: Provision of an aqueous acetate buffer solution as a solvent

In a fifteenth container, 50 µL of an aqueous acetate buffer solution were provided as a solvent with the following dissolved substances at the following concentrations:

| | |
|---|---|
| Potassium acetate | 100 mM |
| Magnesium acetate | 14 mM |
| Tris acetate | 35 mM |
| detergent | 0.45 wt.-% (with regard to the total mass of the aqueous acetate buffer solution) A suitable detergent is NP-40 (nonyl phenoxypol-yethoxylethanol, CAS Registry Number 9016-45-9) |

### 1-3: dissolution of the pellet in the aqueous acetate buffer solution as a solvent

The first pellet (produced in 1-1 of this example) was contacted at 23 °C in the fifteenth container with the 50 µL of the aqueous acetate buffer solution (solvent) already present at 23 °C in the fifteenth container, so that the pellet dissolved in the aqueous acetate buffer solution (solvent) and so that a solution of the pellet ingredients in the aqueous acetate buffer solution resulted.

From the start of the contacting of the first pellet with the aqueous acetate buffer solution until the resulting of the solution (solution of the pellet ingredients in the aqueous acetate buffer solution) no purposeful input of mechanical energy took place; that means that for example no stirring, no shaking and other means of mechanically assisting dissolution like ultrasound etc. were applied.

Within the resulting solution of the pellet ingredients in the aqueous acetate buffer solution, the protein concentrations were those given in Table 1:

**Table 1: Protein concentrations in the solution of the pellet ingredients in the aqueous acetate buffer solution**

| *Protein* | *ng*/*µl* |
|---|---|
| *UvsX* | *300* |
| *UvsY* | *50* |
| *Rb69 Gp32* | *560* |
| *Creatin-kinase (CK)* | *50* |
| *Bsu polymerase* | *96* |
| *Exonuclease* | *80* |

To this solution, a suitable set of primers, a suitable detection probe and a sample comprising a suitable target sequence were added; the resulting solution of the pellet ingredients in the aqueous acetate buffer solution (including primers, detection probe and target sequence) was a sample for use in Recombinase Polymerase Amplification (RPA).

### 1-4: dissolution of the pellet in the aqueous acetate buffer solution as a solvent

The sample produced in above Example 1-3 was used to run RPA reactions in an Axxin T8 Isothermal Instrument and good performance was observed.

### 1-5: Repeatability

Examples 1-1 to 1-4 were repeated ten times in the exact same manner as described. The RPA results showed good repeatability.

### Comparative Example P

### P-1: Manufacture of pellet

In a sixteenth container a defined amount of polyethylene glycol (PEG10000), was provided.

In a seventeenth container, a defined amount of a RB69 GP32 single strand binding protein (https://www.uniprot.orq/uniprot/P09797) with a molecular mass of 33.5 kDa was provided.

In a eighteenth container, a defined amount of a *B*. *subtillis* strand displacement polymerase (BSU https://www.uniprot.orq/uniprot/O34996; Truncated version lacking 5'→3' exonuclease activity) with a molecular mass of 99 kDa was provided.

In a nineteenth container, a defined amount of a T6 Recombinase (UvsX H66S; https://www.uniprot.org/uniprot/A0A346FJC7; Mutation at position 66 (H→S)) with a molecular mass of 44.1 kDa was provided.

In a twentieth container, a defined amount of a T4 Coenzyme of Recombinase (UsvY; https://www.uniprot.orq/uniprot/P04537) with a molecular mass of 15.8 kDa was provided.

In a twenty-first container, a defined amount of E.coli Exonuclease III (Exo III; https://www.uniprot.orq/uniprot/P09030) with a molecular mass of 30.9 kDa was provided.

In a twenty-second container, a defined amount of creatine kinase (CK (Bovine) type B; https://www.uniprot.org/uniprotkb?query=Creatine+Kinase) with a molecular mass of 42.7 kDa was provided.

In a twenty-third container, a defined amount of adenosine triphosphate (ATP) was provided.

In a twenty-fourth container, a defined amount of deoxynucleotide triphosphate (dNTP) was provided.

In a twenty-fifth container, a defined amount of creatine phosphate (CP) was provided.

In a twenty-sixth container, a defined amount of dithiothreitol (DTT) was provided.

The amounts provided in the sixteenth, seventeenth, eighteenth, nineteenth, twentieth, twenty-first, twenty-second, twenty-third, twenty-fourth, twenty-fifth and twenty-sixth container were placed together in an twenty-seventh container and thoroughly intermixed in the twenty-seventh container so that a mixture comprising polyethylene glycol (PEG10000) resulted and said mixture contained an amount of 7 wt.-% of polyethylene glycol (PEG10000) with regard to the total mass of said mixture.

A subset of said mixture was pelletized so that a first comparative pellet with a mass of 7 mg resulted, said first comparative pellet contained an amount of 7 wt.-% of polyethylene glycol (PEG10000) with regard to the total mass of said first comparative pellet.

### P-2: Provision of an aqueous acetate buffer solution as a solvent

In a twenty-eighth container, 50 µL of an aqueous acetate buffer solution were provided as a solvent with the following dissolved substances at the following concentrations:

| | |
|---|---|
| Potassium acetate | 100 mM |
| Magnesium acetate | 14 mM |
| Tris acetate | 35 mM |
| detergent | 0.45 wt.-% (with regard to the total mass of the aqueous acetate buffer solution) |

### P-3: dissolution of the pellet in the aqueous acetate buffer solution as a solvent

The first comparative pellet (produced in P-1 of this comparative example) was contacted at 23 °C in the twenty-eighth container with the 50 µL of the aqueous acetate buffer solution (solvent) already present at 23 °C in the twenty-eighth container, so that the comparative pellet dissolved in the aqueous acetate buffer solution (solvent) and so that a solution of the comparative pellet ingredients in the aqueous acetate buffer solution resulted.

From the start of the contacting of the first comparative pellet with the aqueous acetate buffer solution until the resulting of the solution (solution of the comparative pellet ingredients in the aqueous acetate buffer solution) no purposeful input of mechanical energy took place; that means that for example no stirring, no shaking and other means of mechanically assisting dissolution like ultrasound etc. were applied.

Within the resulting solution of the comparative pellet ingredients in the aqueous acetate buffer solution, the protein concentrations were those given in Table 2:

**Table 2: Protein concentrations in the solution of the comparative pellet ingredients in the aqueous acetate buffer solution**

| *Protein* | *ng*/*µl* |
|---|---|
| *UvsX* | *300* |
| *UvsY* | *50* |
| *Rb69 Gp32* | *560* |
| *Creatin-kinase (CK)* | *50* |
| *Bsu polymerase* | *96* |
| *Exonuclease* | *80* |

To this solution, a suitable set of primers, a suitable detection probe and a sample comprising a suitable target sequence were added; the resulting solution of the comparative pellet ingredients in the aqueous acetate buffer solution (including primers, detection probe and target sequence) was a sample for use in Recombinase Polymerase Amplification (RPA).

This solution of the comparative pellet ingredients in the aqueous acetate buffer solution was a comparative sample for use in Recombinase Polymerase Amplification (RPA).

### P-4: dissolution of the pellet in the aqueous acetate buffer solution as a solvent

The comparative sample produced in P-3 (comparative example) was used to run RPA reactions in an Axxin T8 Isothermal Instrument and poor performance was observed.

### P-5: Repeatability

Comparative examples P-1 to P-4 were repeated ten times in the exact same manner as described. The RPA results showed poor repeatability.

### Example 3 - Comparison of turbidity

A pellet "A" with a mass of 7 mg was prepared according to 1-1 of example 1.

A comparative pellet "B" with a mass of 7 mg was prepared according to P-1 of comparative example P.

In a twenty-ninth container, 50 µL of an aqueous acetate buffer solution as defined in 1-2 of example 1 were provided.

In a thirtieth container, 50 µL of an aqueous acetate buffer solution as defined in P-2 of comparative example P were provided.

### 3.1 Determination of turbidity of pellet "A"

Pellet "A" (of example 3) was contacted at 23 °C in the twenty-ninth container with the 50 µL of the aqueous acetate buffer solution (solvent) already present at 23 °C in the twenty-ninth container, so that pellet "A" dissolved in the aqueous acetate buffer solution (solvent) and so that a solution of the pellet (pellet "A") ingredients in the aqueous acetate buffer solution resulted.

From the start of the contacting of pellet "A" with the aqueous acetate buffer solution until the resulting of the solution (solution of the pellet (pellet "A") ingredients in the aqueous acetate buffer solution) no purposeful input of mechanical energy took place; that means that for example no stirring, no shaking and other means of mechanically assisting dissolution like ultrasound etc. were applied.

Within the resulting solution of the pellet (pellet "A") ingredients in the aqueous acetate buffer solution, the protein concentrations were those given in Table 3:

**Table 3: Protein concentrations in the solution of the pellet (pellet "A") ingredients in the aqueous acetate buffer solution**

| *Protein* | *ng*/*µl* |
|---|---|
| *UvsX* | *300* |
| *UvsY* | *50* |
| *Rb69 Gp32* | *560* |
| *Creatin-kinase (CK)* | *50* |
| *Bsu polymerase* | *96* |
| *Exonuclease* | *80* |

The solution of the pellet (pellet "A") ingredients in the aqueous acetate buffer solution was stored for 20 minutes in the dark at 23°C. Thereafter, the solution contained no suspended matter visible to the unaided eye and showed no sign of turbidity, visible to the unaided eye.

### 3.2 Determination of turbidity of comparative pellet "B"

Comparative Pellet "B" (of example 3) was contacted at 23 °C in the thirtieth container with the 50 µL of the aqueous acetate buffer solution (solvent) already present at 23 °C in the thirtieth container, so that comparative pellet "B" dissolved in the aqueous acetate buffer solution (solvent) and so that a solution of the comparative pellet (comparative pellet "B") ingredients in the aqueous acetate buffer solution resulted.

From the start of the contacting of comparative pellet "B" with the aqueous acetate buffer solution until the resulting of the solution (solution of the comparative pellet (comparative pellet "B") ingredients in the aqueous acetate buffer solution) no purposeful input of mechanical energy took place; that means that for example no stirring, no shaking and other means of mechanically assisting dissolution like ultrasound etc. were applied.

Within the resulting solution of the comparative pellet (comparative pellet "B") ingredients in the aqueous acetate buffer solution, the protein concentrations were those given in Table 4:

**Table 4: Protein concentrations in the solution of the comparative pellet (comparative pellet "B") ingredients in the aqueous acetate buffer solution**

| *Protein* | *ng*/*µl* |
|---|---|
| *UvsX* | *300* |
| *UvsY* | *50* |
| *Rb69 Gp32* | *560* |
| *Creatin-kinase (CK)* | *50* |
| *Bsu polymerase* | *96* |
| *Exonuclease* | *80* |

The solution of the comparative pellet (comparative pellet "B") ingredients in the aqueous acetate buffer solution was stored for 20 minutes in the dark at 23°C. Thereafter, the solution contained suspended matter visible to the unaided eye and showed signs of turbidity, visible to the unaided eye.

## Claims

1. Pellet, comprising:
- a first species of enzyme
and
- a polymeric species comprising a chemical structural element defined by the following formula (I): 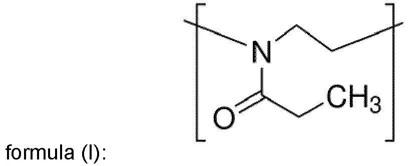
- wherein the polymeric species comprising a chemical structural element defined by formula (I) is poly-(2-ethyl-2-oxazolin);
and
- wherein the pellet contains poly-(2-ethyl-2-oxazolin) in an amount of 3 wt.-%to 8.5 wt.-%, with respect to the total dry mass of the pellet;
and
- wherein the pellet contains poly-(2-ethyl-2-oxazolin) and
- wherein at least 90 % of the poly-(2-ethyl-2-oxazolin) molecules in the pellet have an average relative molecular mass in the range of 10 000 Dalton (Da) to 100 000 Dalton (Da).

2. Pellet according to claim 1,
pellet
- wherein the pellet comprises one, two, three or more additional species of enzyme;
and/or
- wherein the pellet additionally comprises adenosine triphosphate (ATP)
and/or
- wherein the pellet additionally comprises deoxynucleotide triphosphate (dNTP) and/or dideoxynucleotide triphosphate (ddNTP);
and/or
- wherein the pellet additionally comprises dithiothreitol (DTT)
and/or
- wherein the pellet additionally comprises creatine phosphate (CP)
and/or
- wherein the pellet contains poly-(2-ethyl-2-oxazolin) in an amount of from 6 wt.-% to 8.0 wt.-%, preferably from 6.5 wt.-% to 7.5 wt.-%, more preferably in an amount of 7 wt.-%, with respect to the total dry mass of the pellet;
and/or
- wherein the pellet contains poly-(2-ethyl-2-oxazolin) and
- wherein at least 92 % of the poly-(2-ethyl-2-oxazolin) molecules in the pellet, preferably at least 95 % have an average relative molecular mass in the range of 15 000 Da (Dalton) to 80 000 Da (Dalton), more preferably in the range of 20 000 Da (Dalton) to 70 000 Da (Dalton).

3. Pellet according to claim 2, wherein the one, two, three or more different species of enzyme are selected from the group consisting of:
- enzymes for recombinase-polymerase-amplification, including a recombinase, a single-stranded DNA-binding protein (SSB) and a strand-displacing polymerase,
and, optionally, in addition at least one of the following enzymes: reverse transkriptase, creatine kinase, exonuclease III and endonuclease IV.

4. Pellet according to any of the preceding claims, wherein
- the pellet does not have an extension of more than 20 mm, preferably more than 15 mm, more preferably more than 12 mm, even more preferably more than 10 mm in any direction of space;
and/or
- the pellet does not have an extension of less than 4.6 mm, preferably less than 5 mm, more preferably less than 7 mm, even more preferably less than 9 mm in any direction of space;
and/or
- the pellet has a weight in the range of 1 mg to 10 mg, preferably 2 mg to 8 mg, more preferably 5.5 mg to 7.8 mg, even more preferably 6.5 mg to 7.5 mg, yet more preferably 6.8 mg to 7.2 mg;
and/or
- the pellet has a volume in the range of 1 µL to 100 µL, preferably 5 µL to 70 µL, more preferably 10 µL to 50 µL.

5. Pellet according to any of the preceding claims,
- wherein the pellet is completely soluble in tetrahydrofuran, determined according to example B;
and/or
- wherein the pellet is essentially free of polyethylene glycol, preferably completely free of polyethylene glycol;
and/or
- wherein the pellet is essentially free of methylcellulose and/or carboxy-methylcellulose, preferably completely free of methylcellulose and/or carboxy-methylcellulose;
and/or
- wherein the pellet is essentially free of starch, preferably completely free of starch;
and/or
- wherein the pellet is essentially free of alginates, in particular sodium alginate, preferably completely free of alginates, in particular sodium alginate;
and/or
- wherein the pellet is essentially free of soja-polysaccharide, preferably completely free of soja-polysaccharide;
and/or
- wherein the pellet is essentially free of poly methacrylic acid gel, prefer-ably completely free of poly methacrylic acid gel;
and/or
- wherein the pellet is essentially free of Poly[1-(4-sulfophenyl)ethylen], prefer-ably completely free of Poly[1-(4-sulfophenyl)ethylen];
and/or
- wherein the pellet is essentially free of bicarbonates, prefer-ably completely free of bicarbonates;
and/or
- wherein the pellet is essentially free of substances that produce gaseous substances upon contact with acetate buffer solutions;
and/or, preferably "and"
- wherein the pellet is essentially free of microgels, preferably completely free of microgels.

6. Method for detecting a target analyte comprising the following steps:
| | |
|---|---|
| M1) | providing a pellet as defined in any of the preceding claims 1 to 5; |
| M2) | providing an acetate buffer; |
| M3) | providing, preferably as ingredient of the pellet, at least one single-strand binding protein, at least one, and preferably at least two target sequence specific primers, at least one DNA polymerase, and optionally: providing at least one reverse transcriptase; |
| M4) | providing at least one biological sample to be analysed for the presence of the at least one target sequence, and optionally providing an extraction kit, preferably a sterile extraction kit, to obtain the biological sample; |
| M5) | optionally: providing at least one probe and optionally providing, preferably as constituent of the pellet, at least one enzyme activating the probe so that a detectable signal is generated; |
| M6) | dissolving the pellet provided in step M1) in the acetate buffer solution provided in step M2) so that a solution of the ingredients of the pellet in the acetate buffer solution results; |
| M7) | initiating the amplification reaction by adding at least one starting reagent, preferably wherein the starting reagent comprises at least one metal oxide, more preferably at least one metal oxide nano-particle, including zinc oxide, and/or by contacting the components of (a), (b) and optionally (c); and |
| M8) | obtaining the at least one amplified target sequence and/or obtaining at least one detectable signal each signal being indicative of a successful amplification of the respective target sequence amplified. |

7. Method according to claim 6,
- wherein the method for detecting a target analyte is an isothermal method of nucleic acid amplification of at least one target sequence, preferably in a biological sample;
and/or
- wherein the solution of the ingredients of the pellet in the acetate buffer solution resulting in step M6) contains an amount of poly-(2-ethyl-2-oxazolin) in the range of 30 vol.-% to 95 vol.-%, preferably 40 vol.-% to 92 vol.-%, more preferably 50 vol.-% to 90 vol.-%, with regard to the total volume of the solution;
and/or
- wherein the solution of the ingredients of the pellet in the acetate buffer solution resulting in step M6) contains adenosine triphosphate (ATP) in a concentration in the range of 1 mM to 4 mM, preferably 2 mM to 3 mM, more preferably 2.2 mM to 2.8 mM, yet more preferably 2.3 mM to 2.6 mM, even more preferably in a concentration of 2.5 mM;
and/or
- wherein the solution of the ingredients of the pellet in the acetate buffer solution resulting in step M6) contains deoxynucleotide triphosphate (dNTP) and/or dideoxynucleotide triphosphate (ddNTP) in a concentration in the range of 100 µM to 800 µM, preferably 250 µM to 600 µM, more preferably 350 µM to 550 µM, yet more preferably 400 µM to 500 µM, even more preferably in a concentration of 450 µM;
and/or
- wherein the solution of the ingredients of the pellet in the acetate buffer solution resulting in step M6) contains dithiothreitol (DTT) in a concentration in the range of 1 mM to 3 mM, preferably 1.5 mM to 2.7 mM, more preferably 1.6 mM to 2.5 mM, yet more preferably 1.8 mM to 2.2 mM, even more preferably in a concentration of 2 mM;
and/or
- wherein the solution of the ingredients of the pellet in the acetate buffer solution resulting in step M6) contains creatine phosphate (CP) in a concentration in the range of 10 mM to 100 mM, preferably 20 mM to 70 mM, more preferably 40 mM to 60 mM, yet more preferably 45 mM to 55 mM, even more preferably in a concentration of 50 mM.

8. Use of a pellet as defined in any of the preceding claims 1 to 5, in a method according to any of the preceding claims 6 or 7.

9. Use of poly-(2-ethyl-2-oxazolin) as a disintegrating agent in a pellet, preferably in a pellet as defined in any of the preceding claims 1 to 5.

10. Use of poly-(2-ethyl-2-oxazolin) for manufacturing a pellet, preferably a pellet as defined in any of the preceding claims 1 to 5.

11. Use of a pellet as defined in any of the preceding claims 1 to 5, for producing a solution,
- wherein the resulting solution contains an amount of poly-(2-ethyl-2-oxa-zolin) in the range of 30 vol.-% to 95 vol.-%, preferably 40 vol.-% to 92 vol.-%, more preferably 50 vol.-% to 90 vol.-%, with regard to the total volume of the solution;
and/or
- wherein the resulting solution contains adenosine triphosphate (ATP) in a concentration in the range of 1 mM to 4 mM, preferably 2 mM to 3 mM, more preferably 2.2 mM to 2.8 mM, yet more preferably 2.3 mM to 2.6 mM, even more preferably in a concentration of 2.5 mM;
and/or
- wherein the resulting solution contains deoxynucleotide triphosphate (dNTP) in a concentration in the range of 100 µM to 800 µM, preferably 250 µM to 600 µM, more preferably 350 µM to 550 µM, yet more preferably 400 µM to 500 µM, even more preferably in a concentration of 450 µM;
and/or
- wherein the resulting solution contains adenosine triphosphate (ATP) in a concentration in the range of 1 mM to 3 mM, preferably 1.5 mM to 2.7 mM, more preferably 1.6 mM to 2.5 mM, yet more preferably 1.8 mM to 2.2 mM, even more preferably in a concentration of 2 mM.

12. Method for producing a solution
from a pellet as defined in any of the preceding claims 1 to 5, wherein the method comprises the following steps:
S1) Supplying in different containers
- a pellet
- supplying an acetate buffer solution, preferably 10 µL to 100 µL of an acetate buffer solution, more preferably 20 µL to 80 µL, even more preferably 40 µL to 60 µL, yet more preferably 50 µL, as a solvent;
S2) contacting
- the pellet supplied in Step S1)
and
- the solvent supplied in step S1)
in a container so that the pellet supplied in step S1) dissolves in the solvent supplied in step S1) so that a solution results.

13. Method according to claim 12,
- wherein the solvent is an aqueous solution comprising
- potassium acetate, preferably in a concentration in the range of from 50 mM to 200 mM, more preferably 70 mM to 150 mM, even more preferably 90 mM to 120 mM, yet more preferably in a concentration of 95 mM to 105 mM;
and/or
- magnesium acetate, preferably in a concentration in the range of from 5 mM to 30 mM, more preferably 10 mM to 20 mM, even more preferably 11 mM to 17 mM, yet more preferably in a concentration of 13 mM to 15 mM;
and/or
- Tris acetate, preferably in a concentration in the range of from 10 mM to 50 mM, more preferably 30 mM to 40 mM, even more preferably 32 mM to 38 mM, yet more preferably in a concentration of 34 mM to 36 mM;
and/or
- a detergent or a detergent mixture, preferably in a concentration in the range of from 10 mM to 50 mM, more preferably 30 mM to 40 mM, even more preferably 32 mM to 38 mM, yet more preferably in a concentration of 34 mM to 36 mM;
and/or
- polyvinylsulfonic acid (PVSA), preferably in an amount of from 0.0001 wt.-% to 0.005 wt.-%, more preferably 0.0005 wt.-% to 0.003 wt.-%, even more preferably 0.0008 wt.-% to 0.0015 wt.-%, yet more preferably in a concentration of 0.0009 wt.-% to 0.0011 wt.-%;
and/or
- wherein the solvent is essentially free of poly-(2-ethyl-2-oxazolin) and/or polyethylene glycol; preferably completely free of poly-(2-ethyl-2-oxazolin) and/or polyethylene glycol; more preferably essentially free of synthetic polymers;
and/or
- wherein from the start of the contacting in step S2) until the resulting of a solution in step S2) no purposeful input of mechanical energy takes place;
and/or
- wherein from the start of the contacting in step S2) until the resulting of a solution in step S2) the temperature never exceeds 45 °C, preferably never exceeds 38 °C, more preferably never exceeds 30 °C;
and/or
- wherein from the start of the contacting in step S2) until the resulting of a solution in step S2) the temperature is never below 20 °C, preferably never below 18 °C, more preferably never below 15 °C.

14. Use of poly-(2-ethyl-2-oxazolin) to alter enzyme activity, preferably to improve enzyme activity, more preferably to improve enzyme activity in a method according to any of claims 12 to 13.

15. Method for manufacturing a pellet, preferably a pellet according to any of claims 1 to 5, comprising at least the following steps:
G1) providing, preferably providing in separate containers, more preferably in separate containers and in defined amounts,
- at least a first species of poly-(2-ethyl-2-oxazolin)
and
- at least one single-strand binding protein, at least one DNA polymerase, and optionally: at least one reverse transcriptase, together with suitable reaction components,
and/or
- at least one, preferably at least two target sequence specific primers;
and/or
- suitable reaction components, including at least one co-factor for at least one enzyme, dNTPs or a mixture of dNTPs and ddNTPs;
G2) intermixing the materials provided in step G1) within a common container so that a mixture comprising poly-(2-ethyl-2-oxazolin) results;
G3) pelletizing of the mixture comprising poly-(2-ethyl-2-oxazolin) resulting in step G2) so that a pellet results;
and, preferably, comprising the following additional step:
G4) freeze-drying the pellet resulting in step G3 so that a freeze-dried pellet results.

16. Method according to claim 15,
- wherein the molecules of the first species of poly-(2-ethyl-2-oxazolin) provided in step G1) have
- an average relative molecular mass in the range of 25000
and/or
- a polydispersity index in the range of 1 to 2, preferably in the range of 1.2 to 1.6;
or
- wherein the molecules of the first species of poly-(2-ethyl-2-oxazolin) provided in step G1) have
- an average relative molecular mass in the range of 50000 Da and/or
- a polydispersity index in the range of 2 to 5, preferably in the range of 3 to 4;
and/or
- wherein, additionally, in step G1) at least a second species of poly-(2-ethyl-2-oxazolin) is provided, preferably in a separate container and more preferably in a separate container and in a defined amount, and
- wherein the molecules of the second species of poly-(2-ethyl-2-oxazolin) provided in step G1) have an average relative molecular mass
- in the range of 25000 Da
and/or
- a polydispersity index in the range of 1 to 2, preferably in the range of 1.2 to 1.6;
or
- wherein the molecules of the second species of poly-(2-ethyl-2-oxazolin) provided in step G1) have an average relative molecular mass
- in the range of 50000 Da
and/or
- a polydispersity index in the range of 2 to 5, preferably in the range of 3 to 4.

17. Method according to any of the preceding claims 15 or 16,
- wherein
- the molecules of the first species of poly-(2-ethyl-2-oxazolin) provided in step G1) have an average relative molecular mass
- in the range of 25000 Da
and/or
- a polydispersity index in the range of 1 to 2, preferably in the range of 1.2 to 1.6;
and
- wherein, additionally, in step G1) at least a second species of poly-(2-ethyl-2-oxazolin) is provided, preferably in a separate container and more preferably in a separate container and in a defined amount, and
- wherein the molecules of the second species of poly-(2-ethyl-2-oxazolin) provided in step G1) have an average relative molecular mass
- in the range of 50000 Da
and/or
- a polydispersity index in the range of 2 to 5, preferably in the range of 3 to 4;
and/or;
- wherein, additionally, in step G1) at least a second species of poly-(2-ethyl-2-oxazolin) is provided, preferably in a separate container and more preferably in a separate container and in a defined amount, and
- the ratio of
- the total mass of the second species of poly-(2-ethyl-2-oxazolin) provided in step G1) and/or present during intermixing in step G2)
to the
- the total mass of the first species of poly-(2-ethyl-2-oxa-zolin) provided in step G1) and/or present during intermixing in step G2)
is in the range of 10 to 1.2, preferably 8 to 1.4, more preferably the weight ratio is 5.4 : 1.6.

18. Method according to any of the preceding claims 15 to 17, wherein at least two pellets, a first pellet and a second pellet, are produced with the same overall composition, and wherein
the scattering intensity of a solution of the first whole pellet, determined according to steps ES1 and ES2 of example A, does not deviate by more than 30 %, preferably by not more than 20 %, particularly preferably by not more than 10 %, from the average scattering intensity determined from five arbitrarily selected fragments of the second pellet according to steps ES3 to ES5 of example A.

19. Test Kit comprising:
(i) a pellet according to any of claims 1 to 5;
(ii) at least one single-strand binding protein, at least one DNA polymerase, and optionally: at least one reverse transcriptase, together with suitable reaction components,
(iii) at least one, preferably at least two target sequence specific primers;
(iv) suitable reaction components, including at least one co-factor for at least one enzyme, dNTPs or a mixture of dNTPs and ddNTPs, at least one buffer system, and at least one reducing agent;
(v) at least one sterile set for extracting a biological probe to be analyzed and comprising at least one component configured to be applied to a system for analyzing the probe;
wherein preferably the test kit additionally comprises:
(vi) at least one probe and optionally at least one enzyme activating the probe;
and/or
(vii) a second part comprising (i) to (v), preferably (i) to (vi), wherein the second part comprises a predetermined second target sequence acting as positive control and at least one, preferably at least two primers specific for the second target sequence, and preferably a second probe.

20. A use of at least kit as defined in claim 19, for detecting an analyte, preferably for performing an isothermal method of nucleic acid amplification of at least one target sequence.
